# FASCICULE DE BREVET EUROPEEN

(11) **EP 2 167 675 B1**
(45) Date de publication et mention de la délivrance du brevet: **26.10.2011**
(21) Numéro de dépôt: 08826786.9
(22) Date de dépôt: 23.06.2008
(51) Int. Cl.: C12P 19/04, C12P 19/12, C12N 9/88, A61K 8/97, A61K 36/05

(54) **Procédé de clivage enzymatique de polysaccharides issus des algues**
Verfahren zur enzymatischen Spaltung von aus Algen gewonnenen Polysacchariden
Method for enzymatic cleavage of polysaccharides derived from algae

(30) Priorité: 22.06.2007 FR 0704494
(43) Date de publication de la demande: 31.03.2010
(73) Titulaire: Université de Picardie Jules Verne, 80025 Amiens (FR)
(72) Inventeur: EL BOUTACHFAITI, Redouan, 80000 Amiens (FR); PHEULPIN, Patrice, F-94220 Charenton-Le-Pont (FR); COURTOIS, Bernard, F-80090 Amiens (FR); COURTOIS-SAMBOURG, Josiane, F-80090 Amiens (FR)
(74) Mandataire: Bertrand, Didier
(86) Numéro de dépôt international: PCT/FR2008/000881
(87) Numéro de publication internationale: WO 2009/016275

(56) Documents cités:
- LAHAYE M ET AL: "Fine chemical structure analysis of oligosaccharides produced by an ulvan-lyase degradation of the water-soluble cell-wall polysaccharides from Ulva sp. (Ulvales, Chlorophyta)" CARBOHYDRATE RESEARCH, ELSEVIER SCIENTIFIC PUBLISHING COMPANY. AMSTERDAM, NL, vol. 304, no. 3-4, 28 novembre 1997 (1997-11-28), pages 325-333, XP004101618 ISSN: 0008-6215 cité dans la demande
- LAHAYE M: "NMR spectroscopic characterisation of oligosaccharides from two Ulva rigida ulvan samples (Ulvales, Chlorophyta) degraded by a lyase" CARBOHYDRATE RESEARCH, ELSEVIER SCIENTIFIC PUBLISHING COMPANY. AMSTERDAM, NL, vol. 314, no. 1-2, 21 décembre 1998 (1998-12-21), pages 1-12, XP004165157 ISSN: 0008-6215
- LAHAYE M ET AL: "Structure and function properties of Ulvan, a polysaccharide from green seaweeds" BIOMACROMOLECULES 200706 US, [Online] vol. 8, no. 6, juin 2007 (2007-06), pages 1765-1774, XP002522792 ISSN: 1525-7797
- LEBUHN MICHAEL ET AL: "Taxonomic characterization of Ochrobactrum sp. isolates from soil samples and wheat roots, and description of Ochrobactrum tritici sp. nov. and Ochrobactrum grignonense sp. nov" INTERNATIONAL JOURNAL OF SYSTEMATIC AND EVOLUTIONARY MICROBIOLOGY, vol. 50, no. 6, novembre 2000 (2000-11), pages 2207-2223, XP002460123 ISSN: 1466-5026
- MICHAUD P ET AL: "Polysaccharide lyases: Recent developments as biotechnological tools" CRC CRITICAL REVIEWS IN BIOTECHNOLOGY, CRC PRESS, BOCA RATON, FL, US, vol. 23, no. 4, 1 janvier 2003 (2003-01-01), pages 233-266, XP008086252 ISSN: 0738-8551

## Description

La présente invention se rapporte à un procédé de clivage enzymatique de polysaccharides extraits des algues du genre *Ulva.*

Les polysaccharides sont des polymères et ils constituent les éléments essentiels des algues vertes du genre *Ulva.* En outre, ces algues vertes sont très largement répandues sur les côtes de tous les continents et par conséquent largement disponibles à faible coût.

Ces polymères sont dénommés « ulvane » et ils contiennent des oses acides, notamment l'acide glucuronique (Glc*p*A) et l'acide iduronique (Ido*p*A) et aussi des oses neutres dont le rhamnose (Rhap), le galactose (Gal*p*), le glucose (Glc*p*) et le xylose (Xyl*p*). En outre, dans ces polymères, le rhamnose est substitué par un groupement sulfate sur le carbone 3, que l'on dénommera, rhamnose 3 sulfate (Rhap3S).

Ces polymères contiennent des composés intéressants, en particulier l'acide iduronique et le rhamnose 3 sulfate, notamment pour des applications thérapeutiques ou cosmétiques.

Cependant, les oses de ces polymères sont enchaînés de façon relativement aléatoire, de sorte qu'il est malaisé d'extraire une composition relativement homogène. Deux séquences majoritaires, dénommées acides aldobiuroniques, incluant lesdits composés intéressants, ont toutefois été identifiées, une première séquence [→4)-β-D-Glc*p*A-(1→4)-α-L-Rha*p*3 sulfate-(1→]ₙ et une seconde séquence [→4)-α-L-Ido*p*A-(1→4)-α-L-Rha*p*3 sulfate-(1→]ₘ. Des séquences contenant des enchaînement d'acide glucuronique soit : [→4)-β-D-Glc*p*A-(1→4)-β-D-Glc*p*A-(1→]ₓ ont également été décrites ; ces séquences de glucuronane pouvant être intégrées dans les 2 séquences majoritaires constituées des acides aldobiuroniques précités. Cependant, sans que cela ait été prouvé, ces séquences de glucuronane pourraient aussi être présentes sur un polymère différent de l'ulvane, mais extrait de l'algue en même temps que le polymère constitué majoritairement de séquences d'acides aldobiuroniques.

Ces composés sont respectivement comparables aux glycosaminoglycanes, notamment de l'héparine et de l'héparane sulfate, et aux, chondroitine sulfate et dermatane sulfate.

Toutefois, aussi intéressant qu'ils soient, ces polymères extraits des algues vertes, présentent une trop grande hétérogénéité pour pouvoir être utilisés et incorporés tels quels dans des préparations pharmaceutiques ou cosmétiques. Aussi, il a été imaginé de les fractionner en composés élémentaires au moyen de compositions enzymatiques spécifiques.

Ainsi, une première enzyme de la classe des lyases, la glucuronane lyase, a déjà été identifiée, et elle est active pour cliver les séquences de glucuronane présentes dans des polysaccharides de type ulvane. En outre, cette enzyme est obtenue à partir d'un micro-organisme fongique, en particulier de la souche *Trichoderma* CNCM I-3400. On pourra se reporter au document FR 2 885 911, lequel décrit ce micro-organisme pour dégrader les séquences de glucuronane des polysaccharides.

Toutefois, cette composition enzymatique ne permet pas de cliver précisément les séquences majoritaires constituées d'acides aldobiuroniques précitées, dont le fruit présente un intérêt.

Il est néanmoins connu, une autre composition enzymatique, de la classe des lyases, l'ulvane lyase, qui permet, elle, a priori, le clivage des première et seconde séquences d'acides aldobiuroniques précitées au niveau des liaisons osidiques, entre les unités Rhap3 sulfate et Glc*p*A d'une part, et entre les unités Rha*p*3 et Ido*p*A d'autre part, et selon une réaction de β élimination.

Cette composition enzymatique est décrite dans la publication de M. Lahaye et al. (Carbohydrate Research 1997, 304 :325-333) et elle est issue d'une bactérie marine. Elle permet de fractionner les polymères d'ulvane en oligomères.

Cependant, la composition enzymatique décrite dans cette publication et notamment son activité enzymatique par rapport au substrat fait de polymères ulvane est relativement faible, et au surplus, elle décroît rapidement au fur et à mesure de la dégradation, de sorte qu'il est nécessaire de réintroduire des enzymes de ladite composition enzymatique pour que la dégradation se poursuive. En effet, la publication précitée indique que la dégradation de l'ulvane par l'extrait enzymatique isolé est lente et atteint rapidement un plateau. Ainsi il y est décrit, que l'augmentation des sucres réducteurs sur l'ulvane soumis à la dégradation de l'enzyme, augmentation qui est logiquement identique à celle des sucres en position terminale non réductrice, est de 7,1% par rapport au dosage de ces sucres avant l'incubation en présence d'enzyme ; de surcroît, cette dégradation obtenue en 200 minutes nécessite des additions répétées d'enzyme, en moyenne toutes les 25 minutes, pour compenser l'inactivation de l'enzyme par des produits de dégradation. Par conséquent, il n'est pas envisageable d'utiliser cette composition enzymatique, pour produire industriellement des oligomères de compositions et de poids moléculaire homogènes, car le coût d'obtention de ces oligomères serait prohibitif.

Aussi, un problème qui se pose et que vise à résoudre la présente invention est de fournir, selon un premier aspect, un procédé de clivage enzymatique qui permette non seulement de cliver les polymères ulvane entre les unités Rha*p*3 sulfate et Glc*p*A, et entre les unités Rha*p*3 et Ido*p*A, mais aussi qui permette de le faire avec une productivité permettant d'obtenir les oligomères de taille réduite à un coût avantageux.

Dans le but de résoudre ce problème, la présente invention propose un procédé de clivage enzymatique de polysaccharides comprenant une première séquence [→4)-β-D-Glc*p*A-(1→4)-α-L-Rha*p*3 sulfate-(1→]ₙ et une seconde séquence [→4)-α-L-Ido*p*A-(1→4)-α-L-Rha*p*3 sulfate-(1→]ₘ, la première et la seconde séquences étant respectivement constituées de deux unités oses reliées par une liaison osidique, ledit procédé étant du type selon lequel : on fournit lesdites séquences de polysaccharide ; on fournit un micro-organisme apte à produire une substance enzymatique de la classe des lyases ; et, on met en contact ladite substance enzymatique avec lesdites séquences de polysaccharide de manière à provoquer le clivage de la liaison osidique selon une réaction de β élimination, suivant le mécanisme schématique :

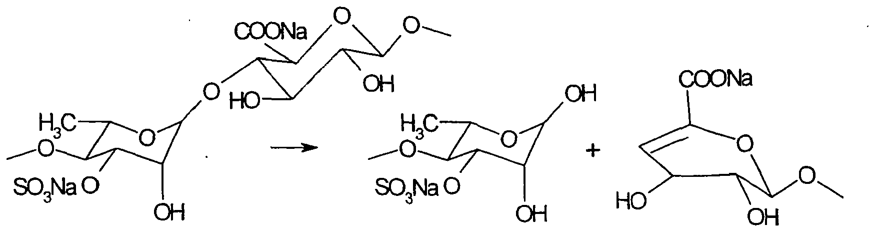

Selon l'invention, on choisit un micro-organisme appartenant aux bactéries du genre *Ochrobactrum* pour produire ladite substance enzymatique.

Ainsi, une caractéristique de l'invention réside dans la mise en évidence de ces bactéries du genre *Ochrobactrum,* qui produisent une substance enzymatique apte à venir couper spécifiquement la liaison osidique par β-élimination sans pour autant entraîner l'apparition de produits de dégradation qui viendraient perturber précisément l'activité enzymatique. Et ce, contrairement aux substances enzymatiques décrites dans l'art antérieur, et issues de bactéries marines, dont l'activité enzymatique est très rapidement interrompue, et pour lesquelles il est alors nécessaire d'introduire dans le milieu réactionnel, de nouvelles substances enzymatiques afin que la dégradation se poursuive.

Au surplus, la β élimination correspond à un clivage de la liaison osidique sur une séquence osidique générant 2 fragments de molécules saccharidiques et faisant apparaître au niveau de l'extrémité terminale non réductrice créée, un hétérocycle présentant une liaison éthylénique entre le carbone 4 et le carbone 5, le résidu de l'extrémité terminale non réductrice correspondant à un acide 4-déoxy-(hex-4-ène) pyranosyluronique. Par ailleurs, comme on l'expliquera ci-après, grâce à l'apparition de cette liaison éthylénique, on mesurera aisément la vitesse de réaction en spectroscopie UV, entre 230 et 240 nm et plus précisément à 235 nm.

Aussi, on observera que le clivage entre les unités Rha*p*3 sulfate et Glc*p*A, et entre les unités Rhap3 et Ido*p*A, conduit exactement aux mêmes produits de réaction, bien que d'un côté, pour la première séquence on ait dans le polymère l'acide glucuronique, tandis que pour la seconde séquence on ait l'acide iduronique. Cela tient à ce que ces deux acides diffèrent simplement par la configuration de leur carbone 5. Ainsi, puisque le carbone 5 est initialement hybridé sp³ et qu'ensuite, il devient hybridé sp², après réaction de β élimination, il n'y a plus qu'une configuration possible.

Ainsi, non seulement la substance enzymatique obtenue grâce à la bactérie du genre *Ochrobactrum* permet d'obtenir des oligomères de faible poids moléculaire avec une plus grande productivité que les substances enzymatiques obtenues avec des bactéries marines, mais au surplus le procédé selon l'invention permet d'obtenir des oligomères homogènes avec une productivité importante.

De façon particulièrement avantageuse, la souche bactérienne choisie appartient à l'espèce *Ochrobactrum tritici* laquelle se retrouve dans le sol. Plus précisément, cette souche bactérienne notée PEC.2 porte la référence CNCM I-3776, dûment déposée et enregistrée auprès de la Collection Nationale de Cultures de Microorganismes (CNCM) au siège de l'institut Pasteur, 25 rue du Docteur Roux, F-75724 Paris cedex 15.

Selon un mode préféré de mise en oeuvre de l'invention, on incube lesdites bactéries avec un substrat polysaccharidique de manière à produire ladite substance enzymatique, et ensuite on isole les bactéries incubées de ladite substance enzymatique afin d'utiliser cette dernière pour produire les oligomères recherchés.

Avantageusement, ladite substance enzymatique est purifiée après que les bactéries ont été incubées, de manière à retenir exclusivement l'enzyme spécifique du clivage de la liaison osidique. Ainsi, l'activité enzymatique de la substance enzymatique qui ne contient alors que l'enzyme, est alors optimale si on la rapporte à la quantité de molécules clivées par minute, et par quantité de ladite substance enzymatique.

Préférentiellement d'ailleurs, la substance enzymatique obtenue grâce la souche bactérienne de l'espèce *Ochrobactrum,* provoque le clivage d'un nombre de liaisons osidiques desdites séquences de polysaccharide compris entre 6.10¹⁶ et 6.10²⁰ par minute et par milligramme de ladite substance enzymatique, par exemple entre 6.10¹⁷ et 6.10¹⁹, et plus précisément encore, 6.10¹⁸. Ainsi qu'on l'expliquera ci-après, cette activité enzymatique correspond sensiblement à 10 U/mg, selon la mesure usuelle; une U d'enzyme représentant ici, la quantité d'extrait enzymatique qui entraîne l'apparition d'une micro-mole d'acide 4-déoxy-(hex-4-ène) pyranosyluronique.

Selon un autre mode préféré de mise en oeuvre de l'invention, lesdits polysaccharides comprenant en outre des séquences de glucuronane à l'intérieur des séquences d'acides aldobiuroniques mais aussi, très probablement du glucuronane sous la forme d'un polymère distinct extrait en même temps que le polymère riche en acides aldobiuroniques, on élimine alors ledit glucuronane. Ainsi, on obtient un polysaccharide contenant essentiellement les deux acides aldobiuroniques et contenant encore les séquences de glucuronane piégées dans les séquences [→4)-β-D-Glc*p*A-(1→4)-α-L-Rha*p*3 sulfate-(1→]ₙ, [→4)-α-L-Ido*p*A-(1→4)-α-L-Rha*p*3 sulfate-(1→]ₘ ; mais le polysaccharide extrait de l'algue est débarrassé du glucuronane libre. Comme on l'expliquera ci-après, grâce à cette séparation on privilégie l'activité de l'ulvane lyase.

Selon encore un autre mode préféré de mise en oeuvre de l'invention lesdits polysaccharides comprennent en outre des héparanes et/ou des héparanes sulfates incluant des polymères constitués de [→4)-β-D-Glc*p*A-2-sulfate-(1 →4)-α-D-GlcNsulfo-6-sulfate-(1→]ₓ et/ou de [→4)-α-L-Ido*p*A-2-sulfate (1→4)-α-D-GlcNsulfo-6-sulfate -(1→]_{y}.

La dégradation du polymère permet alors d'obtenir, selon les quantités d'enzyme employées et la durée d'incubation, des oligomères d'héparane et d'héparane sulfate de petit degré de polymérisation.

Selon un autre aspect, la présente invention concerne une substance enzymatique adaptée au procédé de clivage enzymatique ci-dessus décrit, ladite substance enzymatique étant obtenue à partir d'un micro-organisme appartenant aux bactéries du genre *Ochrobactrum.* Plus précisément ces bactéries appartiennent à l'espèce *Ochrobactrum tritici* dont la souche enregistrée porte la référence CNCM I-3776.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 représente un diagramme montrant une activité enzymatique spécifique de différents éléments d'une substance enzymatique ;
- la Figure 2 montre un graphique illustrant une mesure de l'activité enzymatique de ladite substance enzymatique ;
- la Figure 3 montre deux spectres superposés et correspondant à des produits de dégradation de l'activité enzymatique selon un premier mode de mise en oeuvre, l'un réalisé en chromatographie d'exclusion stérique sur Biogel P2, et l'autre en-dessous, réalisé en spectroscopie de masse ; et,
- la Figure 4 montre un spectre réalisé en chromatographie d'exclusion stérique sur Biogel P6, correspondant à des produits de dégradation de l'activité enzymatique selon un second mode de mise en oeuvre.

De manière avantageuse, on choisira un substrat incluant des séquences de polysaccharides issues d'une algue du genre *Ulva* et dénommé ulvane. Ces séquences de polysaccharide de type ulvane, comprennent notamment, une première séquence [→4)-β-D-Glc*p*A-(1→4)-α-L-Rha*p*3 sulfate-(1→]ₙ et une seconde séquence [→4)-α-L-Ido*p*A-(1→4)-α-L-Rha*p*3 sulfate-(1→]ₘ.

Ces polymères d'ulvane sont appauvris en glucuronane pour optimiser la production de l'activité ulvane lyase. Toutefois, pour certaines applications, on dégradera par voie enzymatique, des polymères d'ulvane contenant du glucuronane.

Avant de décrire ce mode de séparation, on décrira le mode de préparation d'une souche bactérienne de l'espèce *Ochrobactrum tritici* PEC2 et en particulier la souche portant la référence CNCM I-3776, laquelle est apte à produire une substance enzymatique et précisément une ulvane lyase. Cette ulvane lyase est adaptée à provoquer le clivage de la liaison osidique selon une réaction de β élimination, entre des unités Rha*p*3 sulfate et Glc*p*A, et entre des unités Rha*p*3 et Ido*p*A respectivement des première et seconde séquences polysaccharidiques précitées et selon le schéma réactionnel :

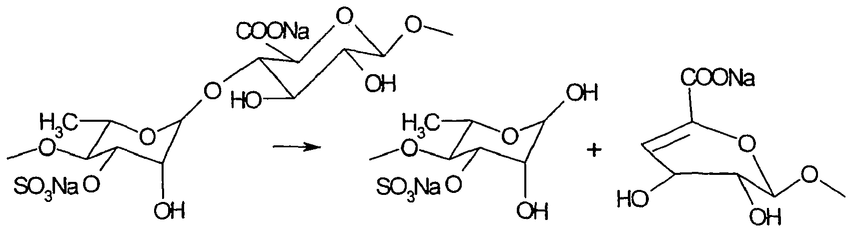

Cette souche est cultivée sur un milieu nutritif minimal minéral contenant : NaCl, 22 g ; Na₂SO₄, 3,7 g ; KCI, 0,6 g ; KBr, 0,1 g ; MgCl₂,6H₂O, 10 g ; CaCl₂,2H₂O, 2,94 g ; NaHCO₃, 0,16 g ; NaNO₃, 25 mg ; NaH₃PO₄, 12H₂O, 5 mg et H₂O QSP 1 L ; le pH étant alors de 7,2. De l'ulvane (2g/L) est ajoutée au milieu nutritif et il constitue alors la seule source de carbone.

La souche apte à dégrader l'ulvane est isolée sur boite de pétri contenant le même milieu nutritif additionné de gélose. Après incubation, on recueille des colonies qui se sont le plus développées. Sont bien évidemment retenues les souches qui métabolisent le substrat en le dégradant selon un mode de β-élimination.

On décrira à présent, le mode de séparation du glucuronane et du substrat ulvane. Pour cela, à partir d'un extrait issu de l'extraction à chaud de polymères de l'ulve et après élimination des insolubles, le pH de l'extrait est abaissé à 2 en présence d'acide dilué. De la sorte, un précipité P incluant des résidus d'acide glucuronique est obtenu et la solution S récupérée, est ramenée à un pH neutre voisin de 7. Cette solution S récupérée, est alors concentrée, purifiée par exemple par ultrafiltration, et ensuite lyophilisée ou précipitée à l'alcool. Ainsi, on obtient un polymère P2 incluant essentiellement des séquences d'acides aldobiuroniques, les séquences de glucuronane restant sont celles intégrées au sein des séquences majoritaires.

S'agissant du précipité P, contenant les résidus d'acide glucuronique, il peut être traité pour obtenir du glucuronane non substitué. Un tel polymère trouve des applications dans de nombreux secteurs industriels.

Avantageusement, le mélange de polymères P2 est utilisé comme substrat pour cultiver la souche d'Ochrobactrum PEC2 et induire la production de l'enzyme « ulvane lyase », laquelle vient couper spécifiquement, par le procédé de β-élimination, d'une part la liaison entre le Rhap3S (le rhamnose 3 sulfate) et le GlcpA (l'acide glucuronique) de la première séquence d'acide aldobiuronique et correspondant à l'enchaînement [→4)-α-L-Rhap3 sulfate-(1→4)-β-D-GlcpA-(1→]ₙ et d'autre part, la liaison entre le Rhap3S (le rhamnose 3 sulfate) et le IdopA (l'acide iduronique) de la seconde séquence d'acide aldobiuronique correspondant à l'enchaînement [→4)-α-L-Rhap3 sulfate-(1→4)-α-L-IdopA-(1→]ₘ.

On décrira à présent, le mode de préparation de l'activité enzymatique. La souche d'Ochrobactrum tritici PEC2 est d'abord inoculée dans un bioréacteur d'une capacité de 2 litres, rempli par 1,5 L de milieu minéral : NaCl 22 g ; Na₂SO₄ 3,7 g ; KCl 0,6g ; KBr 0,1 g ; MgCl₂,7H₂O 5 mg ; NaNO₃ 25 mg ; NaH₃PO₄, 12H₂O, H₂O QSP 1 L, ajusté à pH 7,2 et additionné d'extrait de levure :1g/L, de peptone : 5g/L et de polymères d'ulvane soit préférentiellement de l'ulvane enrichie en acides aldobiuroniques (2g/L).

L'incubation est réalisée préférentiellement entre 25 et 35° C, par exemple 30°C, sous agitation pendant une durée moyenne de 48 heures. Pendant l'incubation, la concentration en ulvane lyase dans le milieu de culture de la souche *Ochrobactrum tritici* PEC2 s'est accrue, et par conséquent l'activité enzymatique du milieu de culture également. Ainsi, le milieu de culture utilisé tel quel permet déjà d'obtenir la dégradation de l'ulvane en oligomères après que la bactérie ait été éliminée par filtration ou centrifugation. Avantageusement, les bactéries sont éliminées du milieu en centrifugeant le milieu de culture à 30 000 g pendant 30 min environ.

On concentre et on purifie l'ulvane lyase selon 3 niveaux de purification. Un premier niveau A de préparation de l'enzyme correspond au milieu de culture débarrassé des bactéries après incubation.

Un second niveau de préparation B est obtenu en additionnant tout d'abord du sulfate d'ammonium (60% p/v) au milieu de culture issu du premier niveau A de préparation pour précipiter les protéines qu'il contient. Les protéines incluent bien évidemment l'enzyme ulvane lyase. Ensuite, on procède à une centrifugation du milieu de culture précipité à raison de 30 000g, pendant 30 minutes et on récupère le culot de protéines. Enfin, le culot de protéines est réintroduit dans environ 10 ml d'une solution tampon Tris/HCl 20 mM à pH 7,5 .

Un troisième niveau de purification C est obtenu en fractionnant par chromatographie sur résine échangeuse d'anions, par exemple sur colonne de DEAE-métacrylate, le fruit de la préparation du second niveau B et en récupérant exclusivement la fraction correspondant à l'ulvane lyase.

S'agissant maintenant de l'activité des préparations enzymatiques elles sont mesurées par la valeur U (unité), laquelle correspond à la quantité d'extrait enzymatique qui entraîne l'apparition d'une micromole (µM) de molécules d'acide 4-déoxy-(hex-4-ène)pyranosyluronique par minute, soit sensiblement 6.10¹⁷ molécules.

L'apparition de ce dernier composé, est mesurée par spectroscopie UV à une longueur d'onde comprise entre 230 et 240 nm et plus précisément à 235 nm, laquelle permet de visualiser l'apparition de la double liaison. Aussi, la valeur d'une unité U correspond à 6.10¹⁷ liaisons osidiques clivées. Par ailleurs, le coefficient d'extinction molaire ε est de 5000 L/ Mole / cm, ce qui permet par application de la loi de Beer-Lambert, après mesure de l'absorption, de calculer les concentrations.

L'activité spécifique correspond, elle, au nombre de µM de molécules d'acide 4-déoxy-(hex-4-ène)pyranosyluronique formées par minute et par milligramme de protéines.

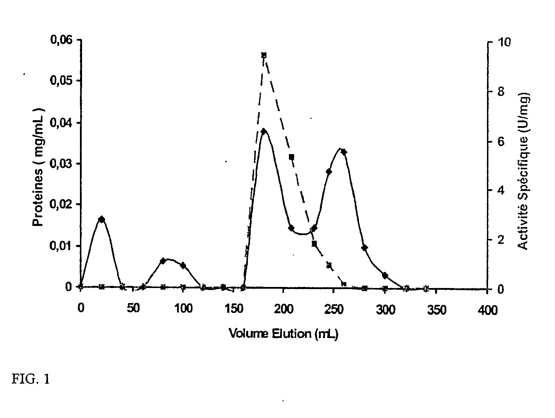

On se reportera à la Figure 1, montrant le profil d'élution en chromatographie échangeuse d'anions sur colonne DEAE-metacrylate semi-préparative, des protéines présentes dans le milieu de culture de la souche *Ochrobactrum tritici* PEC2. L'éluant est du Tris-HCl 0,02 M, à pH=8,5, et à gradient NaCl. Ainsi qu'on l'observe sur cette Figure 1, la courbe en traits continus représentant l'élution des différentes protéines issues du milieu de culture, précipitées au sulfate d'ammonium puis purifiées par chromatographie échangeuse d'anions, et la courbe en traits discontinus, l'activité enzymatique spécifique, on peut aisément recueillir selon le troisième niveau de purification C précité, les enzymes spécifiques de l'activité ulvane lyase. Bien évidemment, le volume d'élution porté en abscisse correspond à un temps de traversée des protéines à travers la colonne, ce temps étant discriminant pour l'enzyme ulvane lyase ; tandis que l'activité spécifique est obtenue par mesure de la absorbance à 235 nm, du produit de l'élution mis en contact avec un substrat ulvane.

Par ailleurs, on reportera dans le tableau ci-dessous, outre l'activité et les caractéristiques des protéines obtenues au troisième niveau C de préparation, celles des protéines obtenues selon les deux autres niveaux de purification A et B.

**Tableau**

| niveaux de purification | Protéines totales (mg) | Activité totale U | Activité spécifique (U/mg) | Rendement % | Rendement % |
|---|---|---|---|---|---|
| Milieu de culture sans bactéries (A) | 130 | 15,65 | 0,12 | 100 | 1 |
| Précipitation au (NH₄)₂SO₄ (B) | 20,76 | 10,7 | 0,515 | 16 | 43 |
| Purification par chromatographie (C) | 0,76 | 7,20 | 9,48 | 0,6 | 80 |

On observera, que l'activité spécifique de la substance ou préparation enzymatique, issu du premier niveau A de préparation et contenant l'ensemble des protéines présentes dans le milieu de culture de la souche *Ochrobactrum* PEC.2, débarrassée des bactéries, est sensiblement 100 fois moins importante que l'activité spécifique de la substance enzymatique incluant exclusivement l'ulvane lyase. Par ailleurs, on notera que le second niveau de purification B permet d'obtenir une activité spécifique près de cinq fois supérieure à l'activité spécifique du milieu d'incubation de la souche d'*Ochrobactrum* débarrassé seulement des bactéries.

Aussi, il convient de calculer les coûts de purification du milieu de culture selon les trois niveaux, afin d'apprécier convenablement la productivité de dégradation des polysaccharides.

### Exemple 1

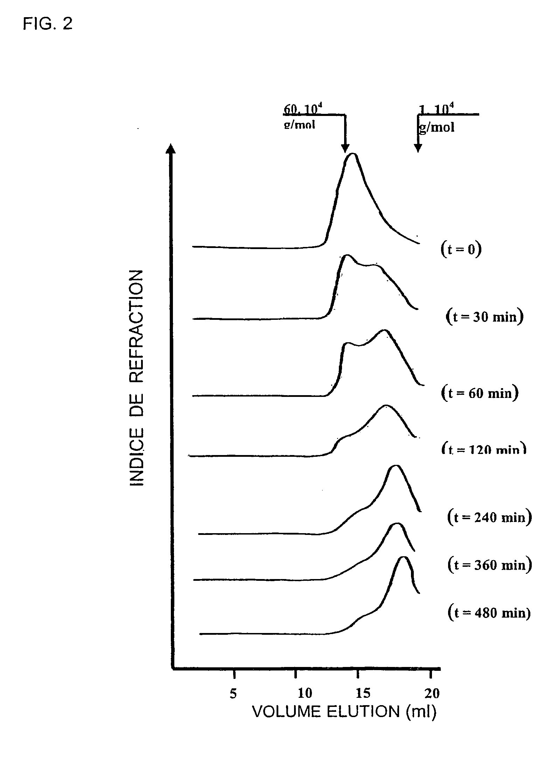

On illustrera dans cet exemple, en référence à la Figure 2, les mesures de vitesse de dégradation enzymatique.

Une préparation enzymatique obtenue selon le troisième niveau de purification C (0,5 U) est appliquée sur 100mL d'une solution de substrat à 2 g/L, le substrat correspondant à de l'ulvane constitué essentiellement d'acides aldobiuroniques. Ce substrat est en effet obtenu en le débarrassant des glucuronanes, selon le procédé décrit plus haut.

La masse moléculaire moyenne de l'ulvane utilisé comme substrat est déterminée par chromatographie d'exclusion stérique couplée à un détecteur à diffusion de lumière en ligne avec un réfractomètre (Technologie SEC-MALLS, pour « Size Exclusion Chromatography Coupled to Multi-angle Laser-Light-Scattering », en langue anglaise) ; cette masse moyenne est de 6.10⁵.

L'expérience de dégradation est réalisée à 30°C pendant une durée totale de 8 heures, et ainsi que le montre la Figure 2, elle est suivie par analyse de la masse moléculaire de l'ulvane par SEC-MALLS, au démarrage de l'expérience, t = 0 ; une demi-heure et une heure après, t = 30 minutes et t = 60 minutes ; deux heures après, t = 120 minutes ; quatre heures après, t = 240 minute ; six heures après, t = 360 minutes et enfin huit heures après, t = 480 minutes.

Au démarrage l'expérience, t = 0, la masse molaire moyenne de l'échantillon est donc de 6.10⁵ g/mol et des fractions de faible masse molaire, sont mises en évidence notamment à partir de l'instant t = 120 min d'incubation. Cela révèle la dégradation du substrat par une activité endoglycosidase.

Après les 8 heures d'incubation, on ne détecte plus d'ulvane de masse élevée de 6.10⁵ g/mol, correspondant au substrat initial.

L'évolution du profil chromatographique selon la technologie SEC-MALLS met en évidence la possibilité d'obtenir des fractions d'ulvane dont la masse moyenne dépendra de la durée d'incubation de l'enzyme en présence du substrat. Selon la durée d'incubation et la concentration en enzyme additionnée en une seule fois au moment de la constitution du mélange réactionnel, on module le degré de polymérisation moyen des oligomères d'ulvane. Pour une incubation de 8 heures, les oligomères obtenus présentent un degré de polymérisation compris essentiellement entre 2 et 25.

### Exemple 2

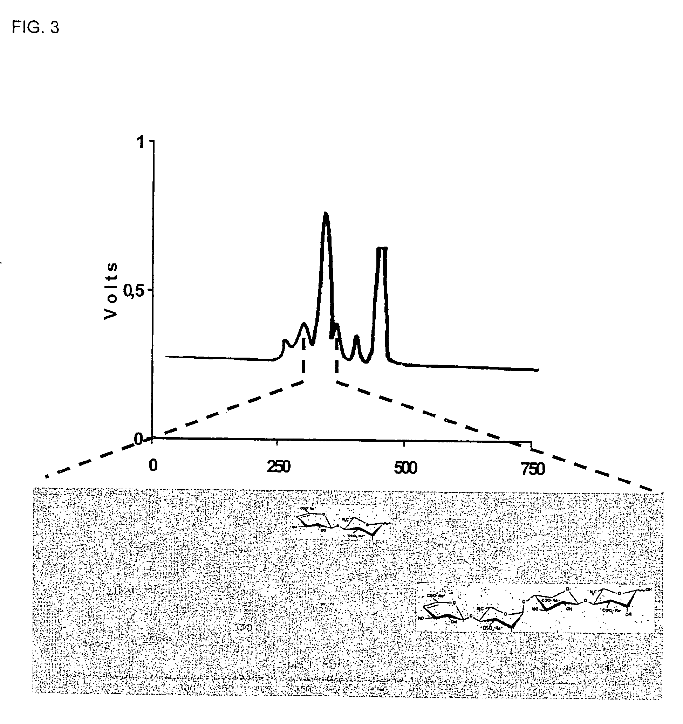

Selon un exemple 2, il est produit, conformément au procédé de clivage enzymatique selon l'invention, des oligomères d'un degré de polymérisation DP essentiellement équivalant à 2. On décrira tout d'abord le mode d'obtention de ces oligomères, et ensuite on se reportera à la Figure 3, illustrant un profil d'élution et en dessous, un spectre de masse correspondant de ces oligomères.

Après une incubation de 72 heures à 30°C d'une préparation de 100 ml constituée d'ulvane enrichi en acides aldobiuroniques à raison de 20g/L, et additionnée de la préparation enzymatique obtenue au troisième niveau de purification C (1 U d'extrait purifié C) en une seule fois au moment de la constitution du mélange, le mélange est chauffé à 100°C, par exemple pendant cinq minutes, pour inactiver l'enzyme. Ensuite, le produit est filtré sur filtre de porosité 0,2 µm pour éliminer les protéines dénaturées. On additionne alors de l'isopropanol (2v) à la préparation pour faire précipiter les composés saccharidiques dont le poids moléculaire est supérieur à 2 000 Da (g/mol) et qui n'auraient pas été dégradés par l'enzyme. Les composés précipités sont récupérés après centrifugation, par exemple à 30 000g pendant 30 minutes, puis ils sont lyophilisés. Le surnageant est soumis à une évaporation pour éliminer l'isopropanol et concentrer l'échantillon qui est lyophilisé à son tour.

Le rendement de la dégradation est obtenu après mesure de la masse des produits présents dans le précipité et dans le surnageant. Dans cet exemple 2, on récupère dans le surnageant 65% d'oligomères d'ulvane de petit degré de polymérisation ; 35% de l'ulvane de départ n'ayant pas été dégradé en petites molécules. L'analyse du produit résultant de la dégradation par chromatographie d'exclusion stérique sur Biogel P2 (détection par réfractométrie) met en évidence une dégradation de 65% du substrat initial en petites molécules. Ainsi que le montre la Figure 3, la fraction majoritaire éluée entre 310 et 380 ml, vers 360 ml est analysée par spectrométrie de masse de type ESI-Q/TOF (pour électrospray-quadrupôle-temps de vol). Les résultats montrent que 80 % des oligomères provenant de la dégradation de l'ulvane présente un degré de polymérisation DP égal à 2, la masse de l'ion étant de 401,1, alors que 20 % des oligomères présente un degré de polymérisation DP égal à 4, la masse de l'ion étant de 824,8.

### Exemple 3

Selon un exemple 3, il est produit des oligomères d'un degré de polymérisation DP essentiellement supérieur à 2.

Pour ce faire, l'incubation de l'enzyme (préparation C, 1 U) en présence de 100 ml du substrat ulvane enrichi en acides aldobiuroniques à raison de 20 g/L, est ici ramenée à 36 heures.

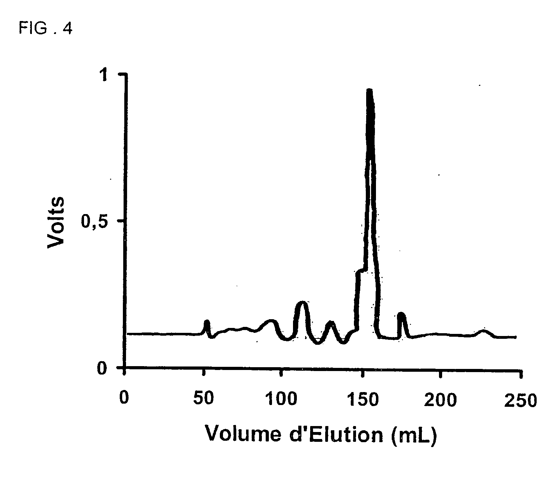

L'élution sur une colonne Biogel P6 met alors en évidence la présence d'oligomères, élués entre 60 et 170 ml, ainsi que l'illustre le profil d'élution représenté sur la Figure 4. On distingue plus précisément des oligomères de petite taille ou d'un plus petit degré de polymérisation DP, élués plus tardivement entre 150 et 170 ml , et aussi des oligomères de plus grande taille d'un degré de polymérisation supérieure à 4, et élués entre 60 et 140 ml. Ainsi, il est mis en évidence ici, la possibilité d'obtenir des oligomères d'ulvane de la taille que l'on souhaite en modulant notamment les paramètres de temps d'incubation et de quantité de substance enzymatique.

Bien entendu, ces résultats obtenus sur des quantités modestes de matières premières, que ce soient de substance enzymatique ou bien de substrat ulvane, peuvent aisément être transposés à des quantités industrielles, dans le but de produire les oligomères désirés à des coûts avantageux.

Dans l'art antérieur, il n'est pas fait état de la production d'oligosaccharides dérivés d'ulvanes enrichis en acides aldobiuroniques. Les d'oligosaccharides selon l'art antérieur ont des degré de polymérisation DP de 2 ou 4 et exceptionnellement 8, car peu d'oligomères sont produits par les procédés décrits et ce sont seuls les majoritaires qui ont été décrits.

S'agissant du procédé selon l'invention, les oligomères sont majoritairement composés d'acides aldobiuroniques car ledit procédé permet d'éliminer le glucuronane présent dans l'extrait brut. Il permet aussi d'ajuster la taille des oligomères souhaités, soit d'un degré de polymérisation DP de 2 ou 4 majoritairement en dégradant pendant 72 h le polymère enrichi en acides aldobiuroniques. Dans ce cas, on obtient un rendement de 65% en oligomères de DP 2 et de DP 4 et 35% du produit résultant de la dégradation présente un degré de polymérisation DP supérieur à 4.

Dans le cas d'une dégradation de 36 h peu d'oligomères de DP 2 sont formés, les oligomères ont majoritairement un DP>4 ainsi que l'illustre la Figure 4. Selon les conditions d'incubation du substrat ulvane en présence de l'enzyme produite par *Ochrobactrum,* il est possible de moduler la taille des oligomères d'ulvane. En 8 h d'incubation le polymère de poids moléculaire moyen de 6 10⁵ Da est dégradé en oligomères de DP majoritairement compris entre 2 et 25.

Il est donc possible par le procédé ici décrit de moduler le DP moyen des oligomères d'ulvane enrichis en acides aldobiuroniques. Par ailleurs, des familles constituées d'oligomères de degré de polymérisation DP plus rapprochés peuvent être obtenues, soit des oligomères de DP 2, 4, ou, des oligomères dont le DP est compris entre 2 et 8 ; ou encore des oligomères dont le DP est compris entre 2 et 25 et entre 4 et 25 par extraction des oligomères de DP égal à 2. Pour ces purifications, on utilisera des procédés de nanofiltration ou des procédés de chromatographie.

Les oligomères obtenus sont ainsi comparables aux glycosaminoglycanes, notamment de l'héparine et de l'héparane sulfate, et aux, chondroitine sulfate et dermatane sulfate. Partant, ils trouvent application dans les domaines thérapeutiques et cosmétiques.

Ainsi, on prévoit des applications des oligosacharides d'ulvane dans le domaine cosmétique, notamment pour traiter la peau contre les lésions causées par l'environnement, l'age et pour protéger et améliorer l'apparence et l'état physiologique de la peau. On prévoit également l'utilisation des oligosacharides d'ulvane pour moduler l'adhésion et la prolifération de cellules tumorales.

On prévoit également des applications dans le domaine phytosanitaire ; par exemple comme agent de fertilisation des plantes. Sont également envisagés, des compositions à partir des familles d'oligomères de degré de polymérisation DP compris entre 2 et 25 et enrichis en acides aldobiuroniques, comme stimulant des défenses naturelles des végétaux contre des stress biotiques et abiotiques.

## Revendications

1. Procédé de clivage enzymatique de polysaccharides comprenant une première séquence [→4)-β-D-Glc*p*A-(1→4)-α-L-Rha*p*3 sulfate-(1→]ₙ et une seconde séquence [→4)-α-L-Ido*p*A-(1→4)-α-L-Rha*p*3 sulfate-(1→]ₘ, la première et la seconde séquences étant respectivement constituées de deux unités oses reliées par une liaison osidique, ledit procédé étant du type selon lequel :
- on fournit lesdites séquences de polysaccharide ;
- on fournit un micro-organisme apte à produire une substance enzymatique de la classe des lyases ; et,
- on met en contact ladite substance enzymatique avec lesdites séquences de polysaccharide de manière à provoquer le clivage de la liaison osidique selon une réaction de β élimination, suivant le mécanisme schématique : 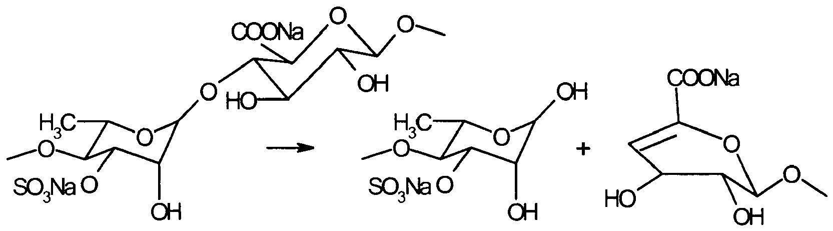
**caractérisé en ce qu'**on choisit un micro-organisme appartenant aux bactéries du genre *Ochrobactrum* pour produire ladite substance enzymatique.

2. Procédé de clivage enzymatique selon la revendication 1, **caractérisé en ce que** la souche bactérienne choisie appartient à l'espèce *Ochrobactrum tritici.*

3. Procédé de clivage enzymatique selon la revendication 1 ou 2, **caractérisé en ce qu'**on incube lesdites bactéries avec un substrat polysaccharidique de manière à produire ladite substance enzymatique, et **en ce qu'**on isole les bactéries incubées de ladite substance enzymatique.

4. Procédé de clivage enzymatique selon la revendication 3, **caractérisé en ce que** ladite substance enzymatique est purifiée pour en retenir l'enzyme spécifique du clivage de la liaison osidique.

5. Procédé de clivage enzymatique selon l'une quelconque des revendications 1 à 4, **caractérisé en ce que** la substance enzymatique provoque le clivage d'un nombre de liaisons osidiques desdites séquences de polysaccharide compris entre 6.10¹⁶ et 6.10²⁰ par minute et par milligramme de ladite substance enzymatique.

6. Procédé de clivage enzymatique selon l'une quelconque des revendications 1 à 5, **caractérisé en ce qu'**on extrait lesdites séquences de polysaccharides à partir d'une algue du genre *Ulva.*

7. Procédé de clivage enzymatique selon la revendication 6, **caractérisé en ce que**, lesdits polysaccharides comprenant en outre des séquences incluant le glucuronane, on élimine ladite séquence incluant le glucuronane, desdites première et seconde séquences [→4)-β-D-Glc*p*A-(1→4)-α-L-Rha*p*3 sulfate-(1 →]ₙ, [→4)-α-L-Ido*p*A-(1→4)-α-L-Rha*p*3 sulfate-(1→]ₘ.

8. Procédé de clivage enzymatique selon la revendication 6 ou 7, **caractérisé en ce que**, lesdits polysaccharides comprennent en outre des héparanes incluant des polymères constitués de [→4)-β-D-Glc*p*A-2-sulfate-(1→4)-α-D-GlcNsulfo-6-sulfate-(1→]ₓ et/ou de [→4)-α-L-Ido*p*A-2-sulfate (1→4)-α-D-GlcNsulfo-6-sulfate -(1→]_{y}.

9. Substance enzymatique de la classe des lyases adaptée au procédé de clivage enzymatique selon l'une quelconque des revendications 1 à 8, **caractérisé en ce qu'**elle est obtenue à partir d'un micro-organisme appartenant aux bactéries du genre *Ochrobactrum.*

10. Substance enzymatique selon la revendication 9, **caractérisée en ce que** la souche bactérienne appartient à l'espèce *Ochrobactrum tritici.*

11. Substance enzymatique selon la revendication 10, **caractérisée en ce que** la souche bactérienne est la souche *Ochrobactrum tritici* CNCM I-3776.

## Claims

1. A method of enzymatic cleavage of polysaccharides comprising a first sequence [→4)-β-D-Glc*p*A-(1→4)-α-L-Rha*p*3 sulfate-(1→]ₙ and a second sequence [→4)-α-L-Ido*p*A-(1→4)-α-L-Rha*p*3 sulfate-(1→]ₘ, the first and the second sequences being respectively constituted of two monosaccharide units joined by a glycoside bond, said method being of the type according to which:
- said polysaccharide sequences are supplied;
- a microorganism able to produce an enzymatic substance of the class of the lyases is supplied; and,
- said enzymatic substance is brought in contact with said polysaccharide sequences so as to cause cleavage of the glycoside bond according to a reaction of β elimination, according to the schematic mechanism: 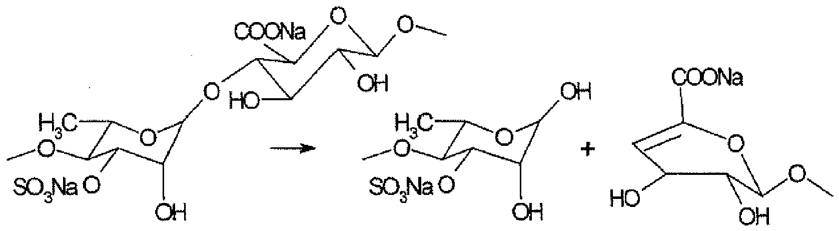
**characterized in that** a microorganism belonging to the bacteria of the genus *Ochrobactrum* is selected for producing said enzymatic substance.

2. The method of enzymatic cleavage as claimed in claim 1, **characterized in that** the bacterial strain selected belongs to the species *Ochrobactrum tritici.*

3. The method of enzymatic cleavage as claimed in claim 1 or 2, **characterized in that** said bacteria are incubated with a polysaccharide substrate so as to produce said enzymatic substance, and **in that** the incubated bacteria are isolated from said enzymatic substance.

4. The method of enzymatic cleavage as claimed in claim 3, **characterized in that** said enzymatic substance is purified so that it retains the enzyme specific to cleavage of the glycoside bond.

5. The method of enzymatic cleavage as claimed in any one of claims 1 to 4, **characterized in that** the enzymatic substance causes the cleavage of a number of glycoside bonds of said polysaccharide sequences between 6.10¹⁶ and 6.10²⁰ per minute and per milligram of said enzymatic substance.

6. The method of enzymatic cleavage as claimed in any one of claims 1 to 5, **characterized in that** said sequences of polysaccharides are extracted from an alga of the genus *Ulva*.

7. The method of enzymatic cleavage as claimed in claim 6, **characterized in that**, with said polysaccharides additionally comprising sequences including glucuronan, said sequence including glucuronan is removed from said first and second sequences [→4)-β-D-Glc*p*A-(1→4)-α-L-Rha*p*3 sulfate-(1→]ₙ, [→4)-α-L-Ido*p*A-(1→4)-α-L-Rha*p*3 sulfate-(1→]ₘ.

8. The method of enzymatic cleavage as claimed in claim 6 or 7, **characterized in that** said polysaccharides further comprise heparans including polymers constituted of [→4)-β-D-Glc*p*A-2-sulfate-(1→4)-α-D-GlcNsulfo-6-sulfate-(1→]ₓ and/or [→4)-α-L-Ido*p*A-2-sulfate (1→4)-α-D-GlcNsulfo-6-sulfate - (1→]_{y}.

9. An enzymatic substance of the class of the lyases suitable for the method of enzymatic cleavage as claimed in any one of claims 1 to 8, **characterized in that** it is obtained from a microorganism belonging to the bacteria of the genus *Ochrobactrum.*

10. The enzymatic substance as claimed in claim 9, **characterized in that** the bacterial strain belongs to the species *Ochrobactrum tritici.*

11. The enzymatic substance as claimed in claim 10, **characterized in that** the bacterial strain is the strain *Ochrobactrum tritici* CNCM 1-3776.

## Patentansprüche

1. Verfahren zur enzymatischen Spaltung von Polysacchariden, die eine erste Sequenz [→4)-β-D-Glc*p*A-(1→4)-α-L-Rha*p*3 sulfat-(1 →]ₙ und eine zweite Sequenz [→4)-α-L-Ido*p*A-(1→4)-α-L-Rha*p*3 sulfat-(1 →]ₘ umfassen, wobei die erste und die zweite Sequenz jeweils aus zwei -ose-Einheiten aufgebaut sind, die durch eine osidische Bindung miteinander verknüpft sind, wobei das Verfahren von dem Typ ist, gemäß dem:
- die Polysaccharidsequenzen bereitgestellt werden;
- ein Mikroorganismus bereitgestellt wird, der geeignet ist, eine enzymatische Substanz der Klasse der Lyasen zu erzeugen; und
- die enzymatische Substanz mit den Polysaccharidsequenzen auf eine Weise in Kontakt gebracht wird, dass die Spaltung der osidischen Bindung durch eine β -Eliminierungsreaktion bewirkt wird, und zwar gemäß dem schematischen Mechanismus: 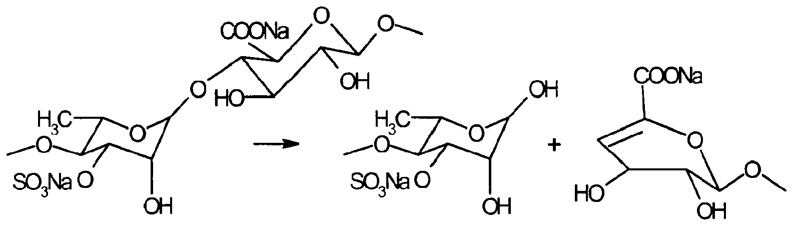
**dadurch gekennzeichnet, dass** ein Mikroorganismus, der zu den Bakterien der Gattung *Ochrobactrum* gehört, zum Herstellen der enzymatischen Substanz ausgewählt wird.

2. Verfahren zur enzymatischen Spaltung nach Anspruch 1, **dadurch gekennzeichnet, dass** der ausgewählte Bakterienstamm zu der Spezies *Ochrobactrum tritici* gehört.

3. Verfahren zur enzymatischen Spaltung nach Anspruch 1 oder 2, **dadurch gekennzeichnet, dass** die Bakterien mit einem Polysaccharidsubstrat auf eine Weise inkubiert werden, dass die enzymatische Substanz erzeugt wird, und dadurch, dass die inkubierten Bakterien von der enzymatischen Substanz isoliert werden.

4. Verfahren zur enzymatischen Spaltung nach Anspruch 3, **dadurch gekennzeichnet, dass** die enzymatische Substanz gereinigt wird, so dass das Enzym, welches spezifisch hinsichtlich der Spaltung der osidischen Bindung ist, zurückgehalten wird.

5. Verfahren zur enzymatischen Spaltung nach einem der Ansprüche 1 bis 4, **dadurch gekennzeichnet, dass** die enzymatische Substanz die Spaltung einer Anzahl osidischer Bindungen der Polysaccharidsequenzen zwischen 6.10¹⁶ und 6.10²⁰ pro Minute und pro Milligramm der enzymatischen Substanz bewi rkt.

6. Verfahren zur enzymatischen Spaltung nach einem der Ansprüche 1 bis 5, **dadurch gekennzeichnet, dass** die Polysaccharidsequenzen aus einer Alge der Gattung *Ulva* extrahiert werden.

7. Verfahren zur enzymatischen Spaltung nach Anspruch 6, **dadurch gekennzeichnet, dass** die Polysaccharide ferner Sequenzen umfassen, die Glucuronan enthalten, und die das Glucuronan enthaltende Sequenz von der ersten und der zweiten Sequenz [→4)-β-D-Glc*p*A-(1→4)-α-L-Rha*p*3 sulfat-(1→]ₙ, [→4)-α-L-Ido*p*A-(1→4)-α-L-Rha*p*3 sulfat-(1→]ₘ eliminiert bzw. entfernt wird.

8. Verfahren zur enzymatischen Spaltung nach Anspruch 6 oder 7, **dadurch gekennzeichnet, dass** die Polysaccharide ferner Heparane umfassen, einschließlich Polymeren, aufgebaut aus [→4)- β -D-Glc*p*A-2-sulfat-(1→4)-α-D-GlcNsulfo-6-sulfat-(1→]ₓ und/oder aus [→4)-α-L-Ido*p*A-2-sulfat (1→4)-α-D-GlcNsulfo-6-sulfat-(1→]_{y}.

9. Enzymatische Substanz der Klasse der Lyasen, die für ein Verfahren zur enzymatischen Spaltung nach einem der Ansprüche 1 bis 8 angepasst ist, **dadurch gekennzeichnet, dass** sie aus einem Mikroorganismus erhalten wird, der zu den Bakterien der Gattung *Ochrobactrum* gehört.

10. Enzymatische Substanz nach Anspruch 9, **dadurch gekennzeichnet, dass** der Bakterienstamm zu der Spezies *Ochrobactrum tritici* gehört.

11. Enzymatische Substanz nach Anspruch 10, **dadurch gekennzeichnet, dass** der Bakterienstamm der Stamm *Ochrobactrum tritici* CNCM I-3776 ist.
